# EUROPÄISCHE PATENTANMELDUNG

(11) **EP 0 988 864 A1**
(43) Veröffentlichungstag der Anmeldung: **29.03.2000**
(21) Anmeldenummer: 99118804.6
(22) Anmeldetag: 23.09.1999
(51) Int. Cl.: A61K 49/00

(54) **Kontrastmittel für die Magnetresonanztomographie**

(30) Priorität: 23.09.1998 CH 194198
(71) Anmelder: Luboldt, Wolfgang, Dr.med. Dipl.-Phys., 45133 Essen (DE)
(72) Erfinder: Luboldt, Wolfgang, Dr.med. Dipl.-Phys., 45133 Essen (DE)
(74) Vertreter: Möbus, Daniela, Dr.-Ing.

(57) **Zusammenfassung**

Ein Kontrastmittel, insbesondere ein endoluminales Kontrastmittel, für die Magnetresonanztomographie, dass mindestens eine ferromagnetische und/oder superparamagnetische und mindestens eine paramagnetische Komponente enthält.

## Beschreibung

Die Erfindung betrifft ein Kontrastmittel, insbesondere ein endoluminales Kontrastmittel, für die Magnetresonanztomographie.

Ein solches Kontrastmittel soll insbesondere eine virtuell endoskopische Darstellung von Hohlräumen sowie eine Kontrastierung des Wassergehaltes und der Perfusion in der umgebenden Begrenzung ermöglichen.

Aus dem Stand der Technik, beispielsweise der EP 0 401 377 B1, sind bereits eisenhaltige Kontrastmittel für die Magnetresonanztomographie zur Kontrastierung von gastrointestinalen Hohlräumen bekannt. Diese bekannten Kontrastmittel erlauben jedoch keine Bildgebung mit den für eine Bildnachverarbeitung, insbesondere die virtuell endoskopische Darstellung von Hohlräumen, erforderlichen Eigenschaften: 1. eine hohe Signalintensität auf maximal T1-gewichteten Aufnahmen, 2. kein oder nur ein geringes Signal auf T2-gewichteten Aufnahmen und 3. eine relativ geringe Signalintensität auf normal T1-gewichteten Aufnahmen. Es ist bekannt, dass mit einer hochkonzentrierten Gadopentatdimeglumin (Gd)-Wasser-Lösung mindestens die ersten beiden Eigenschaften erzielt werden können. Diese Kontrastmittel sind jedoch extrem teuer, sodass sie sich nicht für kolorektale Magnetresonanz-Vorsorgeuntersuchungen eignen.

Der vorliegenden Erfindung liegt die Aufgabe zugrunde, ein kostengünstiges Kontrastmittel zu schaffen, das eine selektive und insbesondere eine virtuell endoskopische Darstellung von Hohlräumen sowie eine Kontrastierung des Wassergehaltes und der Perfusion/Anreicherung intravenös verabreichter Kontrastmittel in der umgebenden Begrenzung ermöglicht.

Die Aufgabe wird erfindungsgemäß durch ein Kontrastmittel, insbesondere ein endoluminales Kontrastmittel für die Magnetresonanztomographie gelöst, dass dadurch gekennzeichnet ist, dass es mindestens eine ferromagnetische und/oder superparamagnetische und mindestens eine paramagnetische Komponente enthält. Es hat sich überraschenderweise gezeigt, dass man beim Zusammenfügen einer oder mehrerer ferromagnetischer bzw. superparamagnetischer und paramagnetischer Komponenten mit einer sehr viel geringeren Gesamtkonzentration dieser Komponenten auskommt als bei Verwendung entweder nur ferromagnetischer oder nur paramagnetischer Komponenten. Dennoch sind die Signaleigenschaften der damit erzeugten Bilder von vergleichbarer oder sogar besserer Qualität. Das erfindungsgemäße Kontrastmittel eignet sich somit zur Herstellung von selektiven dreidimensionalen Aufnahmen kontrastmittelgefüllter Lumina, die auch eine Bildnachverarbeitung, beispielsweise eine virtuelle Endoskopie, erlauben. Ein pathologisch erhöhter Wassergehalt in oder außerhalb der Lumenwand oder flüssigkeitsgefüllte Systeme, wie beispielsweise das Gallengangsystem, sind ebenfalls hervorragend zu kontrastieren. Auch die Kontrastanhebung nach intravenöser Gabe von positiven Kontrastmitteln lässt sich mit einem erfindungsgemäßen Kontrastmittel hervorheben.

Als eine paramagnetische Komponente kommt beispielsweise Gadolinium oder eine gadoliniumhaltige Verbindung wie Gadopentatdimeglumin (Gd-PTPA) in Frage. Es kann jedoch auch Inositolhexaphosphat als paramagnetische Komponente eingesetzt werden. Die paramagnetischen Komponenten bewirken eine Erhöhung der Signalintensität im Vergleich zu Kontrastmitteln mit ausschließlich ferromagnetischen Komponenten. Die Zugabe von paramagnetischen Stoffen kann also helfen, entweder den Kontrast zu maximieren, die für die Rekonstruktion von virtuellen Endoskopien nötige Konzentration an ferromagnetischen Stoffen zur Vermeidung von Nebenwirkungen oder Bildartefakten zu reduzieren oder die Signalintensität auf normal T1-gewichteten Aufnahmen zu minimieren.

Als ferromagnetische oder superparamagnetische Komponenten kommen insbesondere Eisen oder Eisenverbindungen wie Eisenoxide oder Eisenglycerophosphat in Frage. Dabei kann das Kontrastmittel eine Eisenglycerophosphatkonzentration von weniger als 60 g/l und vorzugsweise von weniger als 30 g/l enthalten.

Durch die Zugabe von Zellulose, insbesondere von Methylzellulose, kann die Distension von nicht starren Hohlräumen wie dem Dickdarm sowie die Dauer der lokalen Verweilzeit des Kontrastmittels verbessert werden.

Das erfindungsgemäße Kontrastmittel eignet sich insbesondere auch zur oralen Verabreichung. Insbesondere in Verbindung mit Stuhlweichmachern hat dies den Vorteil, dass Verdauungs- oder Stuhlreste im Darm markiert oder maskiert werden können, sodass Neoplasien besser von Verdauungs- oder Stuhlresten unterschieden werden können und die Intensität der vorherigen Darmreinigung reduziert werden kann. Um bei oraler Verabreichung eine Minderung einer säurebedingten Zersetzung des Kontrastmittels im Magen zu erreichen bzw. die Eisenresorption zu reduzieren, kann das Kontrastmittel außerdem Antazida enthalten.

Besonders gute Bildergebnisse wurden mit Kontrastmitteln mit einer Eisenglycerophosphatkonzentration von 1 bis 30 g/l und einer Dotierung mit 0,5 bis 20 ml/l einer 0,5 molaren gadopentatdimegluminhaltigen Lösung und/oder einer Dotierung mit 1 bis 10 g/l Inositolhexaphosphat bei einer derzeit maximal möglichen T1-Gewichtung mit den Aufnahmeparametern: Repetitionszeit/Echozeit/Anregungswinkel von 4 ms/1.6ms/70° erreicht.

Es wurden damit hintergrundsfreie Aufnahmen eines mit einem erfindungsgemäßen Kontrastmittel gefüllten Dickdarmes mittels dreidimensionaler Gradientenecho-Magnetresonanzsequenzen (spoiled gradient recalled echo (SPGR)/fast low angle shot (FLASH)) erzielt. Diese Aufnahmen eigneten sich hervorragend zur Rekonstruktion einer virtuell endoskopischen Perspektive. Auch eine Kontrastierung der Dickdarmwand mittels T2-gewichteter Sequenzen (z. B. single shot fast spin echo (SSFSE) ist mit diesen Kontrastmitteln einwandfrei gelungen. Durch diese Darstellungsformen lassen sich Anomalien wie Dickdarmpolypen oder Karzinome gut erkennen.

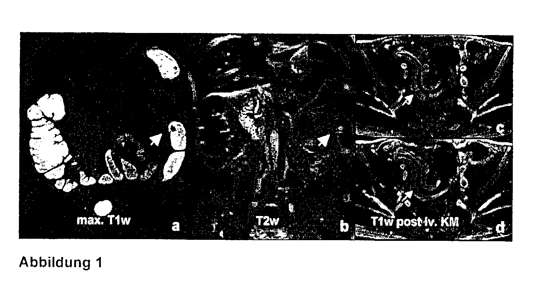

In Abbildung 1 ist eine maximal T1-gewichtete dreidimensionale Aufnahme (a) und T2-gewichtete Aufnahme (b) eines mit Gd-DPTA enthaltenden Kontrastmittel gefüllten Darmes dargestellt. Die Abbildungen 1c und 1d zeigen moderat T1-gewichtete Aufnahmen eines Kontrastmittel gefüllten Darmes nach intravenöser Gabe von positivem Kontrastmittel. Neoplasien, wie der durch einen Pfeil in Abbildung la gekennzeichnete Dickdarmpolyp, werden in der maximal T1-gewichteten Aufnahme als Aussparung im Kontrastmittel sichtbar. Neoplasien können aufgrund ihres Signals auf T2-gewichteten Aufnahmen (Abb. 1b) und ihre Durchblutung auf moderat T1-gewichteten Aufnahmen von Lufteinschlüssen oder Stuhlresten unterschieden werden. Die Durchblutung von Geweben sowie Gefäßleckagen werden durch die intravenöse Gabe von positiven Kontrastmitteln mittels T1-gewichteter Sequenzen dargestellt (Abb. 1c, 1d). Zur besseren Kontrastierung der kontrastmittelperfusion und -anreicherung in der umgebenden Begrenzung sollte das Lumen signalarm sein. Dadurch lassen sich entzündliche Veränderungen, wie beispielsweise beim Morbus Crohn (gekennzeichnet durch Pfeile in den Abb. 1c, 1d), oder Neoplasien gut darstellen.

## Patentansprüche

1. Kontrastmittel, insbesondere endoluminales Kontrastmittel, für die Magnetresonanztomographie, dadurch gekennzeichnet, dass es mindestens eine ferromagnetische und/oder superparamagnetische und mindestens eine paramagnetische Komponente enthält.

2. Kontrastmittel nach Anspruch 1, dadurch gekennzeichnet, dass es mindestens eine Verbindung mit Phosphatgruppen enthält.

3. Kontrastmittel nach Anspruch 2, dadurch gekennzeichnet, dass es Inositolphosphat enthält.

4. Kontrastmittel nach Anspruch 1, dadurch gekennzeichnet, dass es als eine paramagnetische Komponente Gadolinium oder eine gadoliniumhaltige Verbindung enthält.

5. Kontrastmittel nach Anspruch 4, dadurch gekennzeichnet, dass es Gadopentatdimeglumin (Gd-PTPA) enthält.

6. Kontrastmittel nach einem der Ansprüche 1 bis 5, dadurch gekennzeichnet, dass es als eine ferromagnetische oder superparamagnetische Komponente Eisen oder eine Eisenverbindung enthält.

7. Kontrastmittel nach Anspruch 6, dadurch gekennzeichnet, dass es als eine ferromagnetische Komponente Eisenglycerophosphat enthält.

8. Kontrastmittel nach Anspruch 7, dadurch gekennzeichnet, dass es weniger als 60 g/l Eisenglycerophosphat enthält.

9. Kontrastmittel nach einem der Ansprüche 1 bis 8, dadurch gekennzeichnet, dass es außerdem Zellulose enthält.

10. Kontrastmittel nach einem der Ansprüche 1 bis 9, dadurch gekennzeichnet, dass es außerdem Antazida enthält.

11. Kontrastmittel nach einem der Ansprüche 1 bis 10, dadurch gekennzeichnet, dass es außerdem Stuhlweichmacher enthält.
